Europäisches Patentamt

(19) European Patent Office

Office européen des brevets

(11) Numéro de publication: **0 335 835**
**A2**

## (12) DEMANDE DE BREVET EUROPEEN

(21) Numéro de dépôt: 89810221.5

(22) Date de dépôt: 21.03.89

(51) Int. Cl.⁴: **A 61 L 2/00**

(30) Priorité: 29.03.88 CH 1191/88

(43) Date de publication de la demande:
04.10.89 Bulletin 89/40

(84) Etats contractants désignés:
DE FR GB IT NL SE

(71) Demandeur: **ESSILOR INTERNATIONAL**
**1, rue Thomas Edison Echat 902**
**F-94028 Créteil (FR)**

**GALENICA HOLDING S.A.**
**Untermattweg 8**
**CH-3001 Berne (CH)**

(72) Inventeur: **Loretti, Maurice**
**7 ch. du Coin de Terre**
**CH-1219 Châtelaine Genève (CH)**

**Rocklinger, Manfred**
**32 route de Drive**
**CH-1227 Carouge Genève (CH)**

(74) Mandataire: **Vuille, Roman et al**
**c/o KIRKER & Cie S.A. 14, rue du Mont-Blanc Case**
**Postale 872**
**CH-1211 Genève 1 (CH)**

(54) **Dispositif pour le traitement séquentiel d'objets immergés et ensemble comprenant un tel dispositif.**

(57) Le dispositif est destiné au traitement séquentiel d'objets immergés à l'aide de produits réactifs dissous et comprend les éléments suivants :
un réservoir (1) destiné à contenir la solution de produits réactifs,
un réceptacle (2) permettant de maintenir les objets à traiter en immersion dans le réservoir (1),
une pièce de recouvrement (3) fixée au sommet du réservoir (1), supportant en son centre un organe moteur couplé à une minuterie (4), et comportant au voisinage de sa périphérie un orifice (5) permettant le transfert de doses unitaires de produits réactifs à l'intérieur du réservoir (1), et
un distributeur mobile (6) de doses unitaires de produits réactifs centré sur la pièce de recouvrement (3) et prenant appui sur ladite pièce (3), ledit distributeur étant agencé pour être entraîné en rotation par l'organe moteur (4) et comportant au voisinage de sa périphérie des moyens (7) pour amener séparément chacune des doses unitaires de produits réactifs en regard de l'orifice (5) de la pièce (3).

Un tel dispositif peut être entre autres utilisé pour le nettoyage ou le traitement de lentilles de contact.

On décrit également un ensemble constitué du dispositif et d'un récipient de stockage des doses unitaires de produits réactifs.

FIG.1

# Description

## Dispositif pour le traitement séquentiel d'objets immergés et ensemble comprenant un tel dispositif.

L'invention a pour objet un dispositif pour le traitement séquentiel d'objets immergés à l'aide de réactifs dissous. Un tel dispositif est d'un emploi fort diversifié; il se révèle particulièrement bien adapté au traitement de lentilles de contact. L'invention a en outre pour objet un ensemble constitué du dispositif susmentionné et d'un récipient de stockage contenant des doses unitaires de produits réactifs.

Certains objets aussi délicats que les lentilles de contact nécessitent des soins constants pour conserver leurs qualités : nettoyage, désinfection, décontamination ou "déprotéinisation", par exemple. Afin d'éviter une irritation oculaire, l'usager pratique un nettoyage quotidien de ses lentilles de contact. Bien qu'il s'agisse de manipulations relativement simples, elles n'en demeurent pas moins fort délicates, car on utilise des produits réactifs tels que les produits oxydants ou acides, hypochlorite, perborate ou peroxyde d'hydrogène par exemple : le dosage requis doit être très précis et le temps de réaction mesuré avec exactitude sous peine de voir se détériorer les lentilles.

L'ordre des opérations, décontamination, neutralisation, .. doit être bien entendu respecté. Répétées quotidiennement, ces opérations s'avèrent vite fastidieuses et source d'erreurs, sinon d'accidents; elles retiennent en outre l'usager durant plus d'une demi-heure, période au cours de laquelle d'autres activités sont dans la règle à éviter, car elles détournent l'attention de l'usager.

A ce jour, on ne dispose pas d'appareils adéquats permettant d'assurer l'automaticité et la sécurité de telles opérations et l'usager est contraint d'effectuer chaque jour les manipulations décrites ci-dessus, avec tous les risques que cela comporte. Le but de la présente invention est d'offrir à l'usager un dispositif simple, sûr et efficace permettant d'effectuer automatiquement la succession des opérations prévues par le traitement des lentilles de contact, en respectant les doses de produits réactifs et les temps de réaction prescrits.

L'invention parvient au but assigné en proposant un dispositif pour le traitement séquentiel d'objets immergés au moyen de produits réactifs dissous comprenant les éléments constitutifs suivants :
un réservoir 1 destiné à contenir la solution de produits réactifs,
un réceptacle 2 permettant de maintenir les objets à traiter en immersion dans le réservoir 1,
une pièce de recouvrement 3 fixée au sommet du réservoir 1, supportant en son centre un organe moteur couplé à une minuterie 4, et comportant au voisinage de sa périphérie un orifice 5 permettant le transfert de doses unitaires de produits réactifs à l'intérieur du réservoir 1, et
un distributeur mobile 6 de doses unitaires de produits réactifs centré sur la pièce de recouvrement 3 et prenant appui sur ladite pièce 3, ledit distributeur étant agencé pour être entraîné en rotation par l'organe moteur 4 et comportant au voisinage de sa périphérie des moyens 7 pour amener séparément chacune des doses unitaires de produits réactifs en regard de l'orifice 5 de la pièce 3.

Certaines des particularités du dispositif de l'invention apparaîtront à la lecture de la description. Le dispositif ainsi défini permet de surmonter de façon fort avantageuse les inconvénients des techniques appliquées à ce jour pour le traitement des lentilles de contact souples, mais il est évident que son emploi ne se limite pas à ces seules opérations. Dans le traitement des lentilles de contact en particulier, on évite tout contact manuel superflu et toute contamination, l'essentiel du traitement s'effectuant dans un système fermé.

Les dessins annexés illustrent, à titre d'exemples uniquement, certaines exécutions de la présente invention.

Fig. 1 représente, vue en coupe verticale partielle, une première exécution de l'invention.

Fig. 2 représente, vue d'en haut, l'exécution illustrée par Fig. 1.

Fig. 3 est une coupe verticale partielle d'une autre exécution de l'invention.

Fig. 4 représente un ensemble conforme à une exécution de l'invention.

Fig. 5A, 5B, 5C et 5D sont des vues schématiques illustrant le fonctionnement d'une exécution de l'invention.

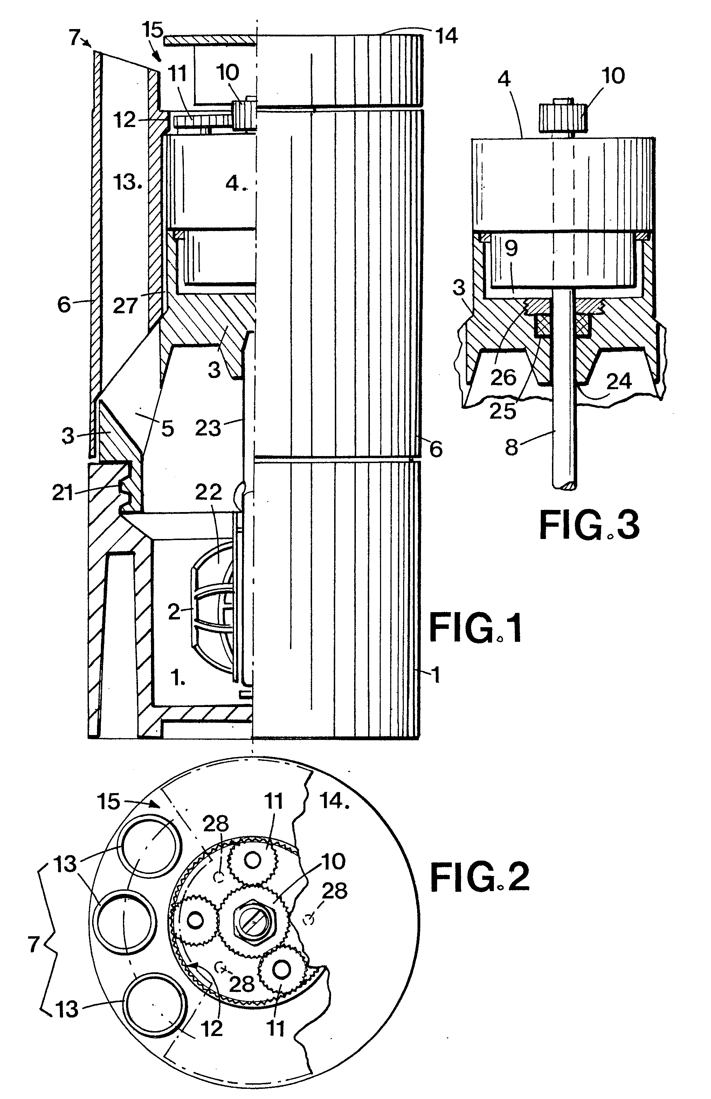

Dans l'une des exécutions de l'invention, le dispositif se présente comme suit : il comprend un réservoir 1, de forme générale cylindrique et de préférence à fond plat, surmonté d'une pièce de recouvrement 3 fixée à la partie supérieure du réservoir. Ladite pièce 3 peut être par exemple vissée au réservoir 1 : dans une telle exécution, le pourtour du réservoir présente un filet 21 coopérant avec celui aménagé à la base de la pièce 3 (Fig. 1). D'autres systèmes de fixation peuvent être bien entendu envisagés, tels des systèmes à crans, à pinces ou à encliquetage par exemple.

Dans une exécution particulière de l'invention, le volume utile du réservoir 1 peut être réduit au minimum. Dans un tel cas son diamètre interne sera sensiblement plus petit que le diamètre de la base du dispositif, ladite base étant par exemple conformée comme illustré par Fig. 1.

Selon l'invention, le dispositif comprend en outre un réceptacle 2, disposé au sein du réservoir 1 et de préférence dans sa partie inférieure. De la sorte, il permet de maintenir en immersion les objets à traiter, une fois le réservoir rempli de la quantité adéquate de liquide. De façon à favoriser le contact des objets à traiter avec les produits réactifs en solution, le réceptacle peut présenter des jours 22 ou toute autre conformation ouverte appropriée, fonction notamment de la nature des objets à traiter. Dans l'une des exécutions de l'invention, le réceptacle 2 est fixé à la surface inférieure de la pièce de recouvrement 3 par l'intermédiaire d'un axe 23 (Fig. 1). Dans un tel cas, le réceptacle reste immobile tant que dure le traitement.

Dans une autre exécution de l'invention, le réceptacle 2 peut être fixé à l'axe 8 de l'organe moteur 4, décrit plus en détail ci-après. Dans cette exécution, l'axe 8 passe au travers d'un passage 24 aménagé au centre de la pièce 3, l'étanchéité étant par exemple assurée au moyen d'un joint torique 25 maintenu dans son logement par une rondelle 26. Le réceptacle peut être ainsi entraîné par le mouvement imprimé à l'axe de l'organe moteur 4, ce qui permet d'activer le mélange des produits réactifs en solution et favorise d'autant le contact des objets en cours de traitement avec ladite solution (Fig. 3).

Conformément à l'invention, la pièce de recouvrement 3 supporte en son centre un organe moteur couplé à une minuterie 4 et présente, au voisinage de sa périphérie, un orifice 5 assurant le transfert de doses unitaires de produits réactifs à l'intérieur du réservoir 1. Dans une exécution particulière de l'invention, la pièce 3 est de forme tronconique et comporte en son centre un logement 9 dans lequel est immobilisé l'organe moteur 4, à l'aide de tout moyen connu (collage, soudage, encliquetage, etc ...). Dans un tel cas, la base du distributeur mobile 6 est bien entendu conformée de manière adéquate, de préférence suivant le même tronc de cône : de la sorte, on obtient une surface de contact des éléments fixe et mobile relativement étendue, répartissant mieux la pression exercée par le distributeur 6 et diminuant d'autant les forces de frottement. La surface de contact de chacun des éléments susmentionnés sera de préférence lisse : les matériaux polymères synthétiques que l'on peut utiliser pour la fabrication autorisent un tel agencement. Une telle disposition est illustrée par Fig. 1.

Bien entendu, l'agencement tronconique décrit ci-dessus n'est pas le seul envisageable et l'on peut tout aussi aisément concevoir une surface de contact plane, perpendiculaire à l'axe du dispositif ou une surface de contact en calotte sphérique p. ex.

Conformément à l'invention, l'organe moteur 4 comprend, de préférence à sa partie supérieure, des moyens d'entraînement 10 solidaires de son axe 8 entraînant, à l'aide de moyens intermédiaires appropriés, le distributeur mobile 6 dans un mouvement de rotation. Dans l'une des exécutions de l'invention telle qu'illustrée par Fig. 1 et 2, les moyens 10 consistent en une roue dentée centrée sur l'axe de l'organe moteur et les moyens intermédiaires susmentionnés consistent en trois roues dentées 11 prenant appui contre la roue 10 et disposées symétriquement, à 120° l'une de l'autre, et coopérant avec des moyens d'entraînement 12 solidaires du distributeur mobile 6, en l'occurrence une couronne dentée.

Par un dimensionnement adéquat des organes 10, 11 et 12 mentionnés ci-dessus, on parvient à répartir uniformément le couple de rotation et à adapter la vitesse de rotation du distributeur mobile 6 aux valeurs requises par sa fonction.

Comme indiqué l'organe moteur 4, non représenté dans le détail, est couplé à une minuterie : il peut s'agir d'un moteur mécanique, électrique (à pile ou batterie) ou électro-mécanique usuel, du moment qu'il peut être adapté aux dimensions du dispositif. Les mécanismes d'entraînement du distributeur mobile, de même que ceux assurant le contrôle de la vitesse de rotation peuvent être, comme on l'a vu, conventionnels tout comme peut l'être la minuterie. Cette dernière peut en outre comprendre un dispositif acoustique, avertissant l'usager de la fin des opérations de traitement, par exemple après une rotation de 360° ou plus selon les cas.

Selon l'invention, le dispositif comprend en outre un distributeur mobile 6 de doses unitaires de produits réactifs centré sur la pièce de recouvrement 3 et, comme on l'a vu précédemment, prenant appui sur ladite pièce 3. Dans l'exécution illustrée par Fig. 1, le distributeur mobile est un cylindre creux, de diamètre externe identique ou quasi identique à celui de la base du réservoir 1 et comportant en son centre un logement 27 aménageant une place suffisante pour l'organe moteur 4. Dans la partie supérieure dudit logement 27 est disposée la couronne dentée 12 décrite précédemment. Au voisinage de sa périphérie, le distributeur mobile 6 comporte des moyens 7 pour amener séparément chacune des doses unitaires de produits réactifs en regard de l'orifice 5 de la pièce 3 : dans ce cas particulier, il s'agit de plusieurs conduits verticaux 13 aménagés dans la masse du cylindre, tous centrés sur un même cercle et bien entendu alignés individuellement sur l'orifice 5 lorsqu'ils sont amenés dans la position requise, au cours de la rotation du distributeur mobile 6. D'autres dispositions sont concevables du moment qu'elles respectent les conditions de juxtaposition définies ci-dessus.

Si cela est souhaité, le dispositif selon l'invention peut comporter des repères visualisant les positions respectives de ses organes fixes, respectivement mobiles, en particulier celles des moyens 7 et de l'orifice 5 de la pièce de recouvrement 3. A de telles fins, on peut déposer des marques ou index adéquats à la surface externe du distributeur mobile 6 et du réservoir 1 par exemple, ce type de repère permettant aisément d'indiquer une position d'arrêt, respectivement de fonctionnement du dispositif, ou encore une position de chargement des doses, respectivement de traitement des objets immergés.

Dans une exécution particulière de l'invention, le dispositif comporte un couvercle 14, de préférence fixé à la partie supérieure de l'organe 4, par exemple au moyen de tétons 28 disposés symétriquement, en alternance avec les roues dentées 11. Dans une telle exécution, le couvercle 14 présente un dégagement 15 permettant un accès aisé aux conduits 13 (délimitation par ligne en traits-points dans Fig. 2). De la sorte, la portion supérieure de chacun des conduits 13 peut être dégagée pour l'insertion des doses unitaires de produits réactifs (phase de chargement), et par la suite masquée une fois le distributeur mobile 6 entraîné dans son mouvement de rotation (phase de fonctionnement).

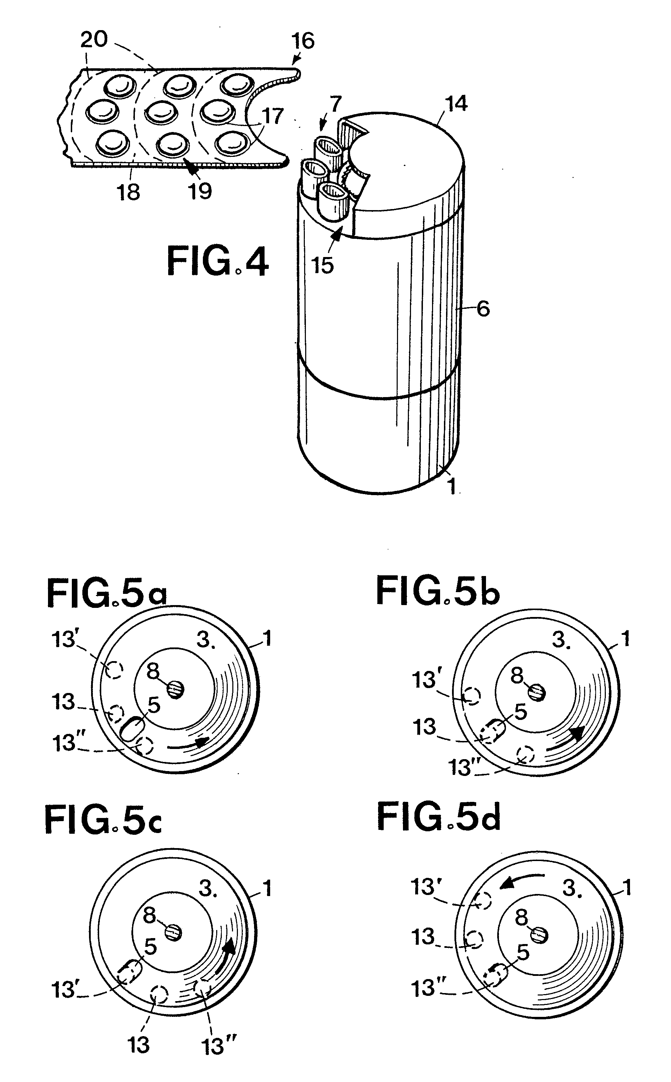

Comme indiqué, l'invention a également pour objet un ensemble constitué du dispositif décrit ci-dessus et d'un récipient de stockage 16 contenant les doses unitaires de produits réactifs. Une exécution particulière d'un tel ensemble est représentée à la Fig. 4.

Selon l'invention, le récipient de stockage 16 comprend une ou plusieurs alvéoles 17 faisant office de réceptacle des doses unitaires de produits réactifs, chacune des alvéoles 17 étant fermée par un opercule déchirable 18. Un tel récipient peut être avantageusement constitué d'une feuille de matériau polymère préformée de matière adéquate, les alvéoles 17 saillant sur l'une de ses faces, une feuille d'aluminium par exemple, étant soudée ou collée sur la face opposée. En regard de chaque alvéole se dessine alors un opercule correspondant 18 que l'on peut déchirer par simple enfoncement de l'alvéole sur le conduit 13.

Dans une exécution particulière de l'ensemble selon l'invention, le récipient de stockage 16 présente plusieurs alvéoles 21 contenant chacune une seule dose unitaire de produit réactif. Dans un tel cas, les alvéoles 17 sont groupées en ensembles 19 (par exemple par groupes de trois) et chaque alvéole de l'ensemble 19 contient une dose unitaire d'un produit réactif distinct. En outre, chacun desdits ensembles 19 est séparé d'un autre par une ligne de déchirure 20. Dans cette exécution particulière, les alvéoles de l'ensemble sont disposées de préférence sur un arc de cercle correspondant à celui défini par les conduits 13. De la sorte, on évite toute confusion quant à l'attribution de la dose unitaire à un conduit spécifique. De même, la ligne de déchirure 20 sera aussi disposée en arc de cercle.

Afin d'illustrer plus en détail la mise en oeuvre des objets de l'invention, on décrira ci-après l'utilisation d'un dispositif destiné au traitement de lentilles de contact souples. Dans un tel cas, les dimensions du dispositif sont de l'ordre de quelques centimètres, l'exécution retenue étant conforme à celle représentée par Fig. 1. Le fonctionnement de ce dispositif est en outre schématiquement illustré à l'aide des Fig. 5A à 5D.

Dans le réservoir 1, on dispose une quantité appropriée de solution physiologique stérile (NaCl à 0,9% dans $H_2O$), puis on immerge le réceptacle 2 contenant les lentilles de contact.

La pièce de recouvrement 3, à laquelle est fixé le réceptacle 2, est alors vissée au réservoir. L'agencement des éléments constitutifs du dispositif est conçu de façon telle que l'usager tienne dans une main l'ensemble formé par ladite pièce 3 supportant l'organe moteur 4, le distributeur mobile 6 et le couvercle 14.

Une fois la pièce 3 parvenue en fin de course, on imprime au distributeur mobile 6 un mouvement de rotation, par exemple de 360° ou plus environ, ce qui a pour effet de remonter le mécanisme de l'organe moteur 4 s'il s'agit d'un mécanisme à ressort. Dans le cas d'un organe moteur électrique ou électro-mécanique, cette opération n'est pas forcément nécessaire.

Lorsque le mécanisme susmentionné est prêt à être mis en marche, l'agencement du dispositif est le suivant, les trois conduits 13, 13′ et 13″ étant suffisamment espacés pour que leur base respective se situe de part et d'autre de l'orifice 5: la base du conduit 13″ se trouve ainsi placée en aval dudit orifice 5, dans le sens de la rotation qui sera imprimée au distributeur mobile par l'organe moteur 4, la base du conduit 13 se situe alors en amont de l'orifice 5 et la base du conduit 13′ en amont de celle du conduit 13. Selon cet agencement en outre, l'extrémité supérieure de ces mêmes conduits débouche dans le dégagement 15 comme illustré par Fig. 4. On dispose alors un ensemble 19 de doses unitaires du récipient de stockage 16 à l'extrémité supérieure desdits conduits 13, 13′ et 13″ puis, après enfoncement de l'opercule, on introduit chacune des doses unitaires prévues dans son conduit respectif (phase de chargement). Cet agencement est illustré par Fig. 5A.

C'est à partir de ce moment que l'usager peut mettre en marche l'organe moteur 4, en libérant par exemple le mécanisme du remontoir. La rotation du distributeur mobile entraîne alors la dose unitaire placée dans le conduit 13 en regard de l'orifice 5, pour son transfert automatique dans le réservoir 1 (Fig. 5B) : cette première dose consiste en un comprimé stable de $NaCl/NaClO_2$. Poursuivant sa rotation, le distributeur entraîne la dose unitaire placée dans le conduit 13′, cette dernière passant automatiquement dans le réservoir 1 au travers de l'orifice 5 (Fig. 5C) : cette seconde dose consiste en un comprimé à base d'acide citrique, dosé en quantité suffisante pour maintenir le pH souhaité au sein du liquide lors du traitement. La dissolution des deux premières doses ainsi introduites dans le liquide permet au produit oxydant d'agir au pH optimum : il s'agit de la phase de nettoyage et de décontamination des lentilles de contact. Poursuivant sa rotation, le distributeur mobile parvient, après un tour quasi complet, dans la position où le conduit 13″ est aligné sur l'orifice 5 (Fig. 5D), ce qui entraîne le transfert automatique de la troisième dose unitaire dans le réservoir 1. Il s'agit dans ce cas d'un comprimé neutralisant à base de perborate de sodium : la phase de neutralisation et stabilisation peut alors démarrer. La vitesse de rotation peut être calculée de façon que la phase de nettoyage dure env. 15 à 20 min. Une fois terminée la période attribuée à la phase de neutralisation, un signal acoustique ou visuel en avertit l'usager et ce dernier peut alors retirer le réceptacle du réservoir et rincer le cas échéant les lentilles ainsi traitées à l'aide d'une nouvelle quantité de solution physiologique stérile. Les lentilles de contact sont alors prêtes à l'usage.

Dans une autre mise en oeuvre du traitement détaillé ci-dessus, on utilisera les produits réactifs suivants :
- première dose (produit oxydant) : dichloroisocyanurate de sodium - deuxième dose (régulation du pH) : sels de type phosphate ou borate de sodium - troisième dose (produit neutralisant) : thiosulfate de sodium.

Dans une autre mise en oeuvre dudit traitement on procédera encore différemment : dans le récipient 1, on introduit une quantité appropriée de peroxyde d'hydrogène à 3% dans l'eau (produit oxydant) en lieu et place de la solution physiologique précitée. Dans un tel cas, il suffira d'introduire la dose de produit réactif servant à la régulation du pH, par exemple du chlorure de sodium, puis la dose de produit neutralisant tel du bisulfite ou du perborate de sodium par exemple.

Le traitement décrit ci-dessus est effectué quotidiennement; à intervalles plus espacés, par exemple hebdomadairement, le traitement de neutralisation peut être complété par un traitement enzymatique, visant à "déprotéiniser" les lentilles. Pour cela, il suffit d'ajouter à la dose unitaire de produit neutralisant, une dose du produit enzymatique approprié qui sera entraînée avec elle par la rotation du distributeur mobile.

Bien entendu, les autres variantes de traitement exposées plus haut peuvent être également mises en oeuvre avec le dispositif.

L'avantage de tels dispositifs est qu'ils autorisent l'emploi de produits réactifs plus concentrés, plus puissants que ceux mis à ce jour à disposition de l'usager, dés lors que les opérations mentionnées plus haut sont automatisées et parfaitement contrôlées dans le temps. On parvient en outre à raccourcir d'autant la période de traitement des lentilles de contact.

Bien entendu, d'autres exécutions du dispositif peuvent être envisagées, en fonction de l'usage auquel on le destine, qu'il s'agisse par exemple du nettoyage de prothèses dentaires, du traitement de pellicules photographiques ou de réactions chimiques ou biochimiques poursuivies à des fins analytiques. Dans de tels cas, les dimen sions du dispositif de l'invention peuvent être nettement supérieures à celles mentionnées plus haut, sans que cela modifie l'agencement particulier de ses divers éléments constitutifs.

**Revendications**

1. Dispositif pour le traitement séquentiel d'objets immergés au moyen de produits réactifs dissous comprenant un réservoir (1) destiné à contenir la solution de produits réactifs,
un réceptacle (2) permettant de maintenir les objets à traiter en immersion dans le réservoir (1),
une pièce de recouvrement (3) fixée au sommet du réservoir (1), supportant en son centre un organe moteur couplé à une minuterie (4), et comportant au voisinage de sa périphérie un orifice (5) permettant le transfert de doses unitaires de produits réactifs à l'intérieur du réservoir (1), et
un distributeur mobile (6) de doses unitaires de produits réactifs centré sur la pièce de recouvrement (3) et prenant appui sur ladite pièce (3), ledit distributeur étant agencé pour être entraîné en rotation par l'organe moteur (4) et comportant au voisinage de sa périphérie des moyens (7) pour amener séparément chacune des doses unitaires de produits réactifs en regard de l'orifice (5) de la pièce (3).

2. Dispositif selon la revendication 1, caractérisé en ce que le réceptacle (2) est solidaire de la pièce de recouvrement (3).

3. Dispositif selon la revendication 1, caractérisé en ce que le réceptacle (2) est solidaire de l'axe (8) de l'organe moteur (4) et entraîné par ledit axe lors de sa rotation.

4. Dispositif selon l'une des revendications 1 à 3, caractérisé en ce que la pièce de recouvrement (3) est de forme tronconique et qu'elle comporte à son sommet un logement (9) destiné à recevoir l'organe moteur (4), la base du distributeur mobile (6) en contact avec la pièce (3) étant conformée suivant le même tronc de cône.

5. Dispositif selon l'une des revendications 1 à 4, caractérisé en ce que l'organe moteur (4) comporte à sa partie supérieure des moyens d'entraînement (10) solidaires de son axe (8) et des moyens de transmission (11) coopérant avec des moyens d'entraînement (12) solidaires du distributeur mobile (6).

6. Dispositif selon la revendication 5, caractérisé en ce que les moyens d'entraînement (10) et de transmission (11) sont des roues dentées et que les moyens d'entraînement (12) du distributeur mobile (6) consistent en une couronne dentée.

7. Dispositif selon l'une des revendications 1 à 6, caractérisé en ce que les moyens (7) consistent en un ou plusieurs conduits (13) aménagés dans la masse du distributeur mobile (6), au voisinage de sa périphérie.

8. Dispositif selon la revendication 7, caractérisé en ce que le distributeur mobile (6) est de forme externe cylindrique et que les moyens (7) consistent en un ou plusieurs conduits (13) verticaux.

9. Dispositif selon l'une des revendications 1 à 8, caractérisé en ce qu'il comporte des repères visualisant les positions respectives de ses organes fixes, respectivement mobiles, en particulier celles des moyens (7) et de l'orifice (5) de la pièce de recouvrement (3).

10. Dispositif selon l'une des revendications 1 à 9, caractérisé en ce qu'il comporte à son sommet un couvercle (14) fixé à la partie supérieure de l'organe moteur (4) et présentant à sa périphérie un dégagement (15) permettant l'accés aux moyens (7).

11. Ensemble constitué du dispositif selon l'une des revendications 1 à 10 et d'un récipient de stockage (16) contenant des doses unitaires de produits réactifs.

12. Ensemble selon la revendication 11, caractérisé en ce que le récipient de stockage (16) comprend une ou plusieurs alvéoles (17) faisant office de réceptacle des doses unitaires de produits réactifs, chacune des alvéoles (17) étant fermée par un opercule déchirable (18).

13. Ensemble selon la revendication 12, caractérisé en ce que le récipient de stockage (16) présente plusieurs alvéoles (18) groupées en ensembles (19), chacune des alvéoles d'un ensemble (19) contenant une seule dose unitaire d'un produit réactif distinct.

14. Ensemble selon la revendication 13, caractérisé en ce que chaque ensemble (19) est séparé de l'autre par une ligne de déchirure (20).

FIG.1

FIG.2

FIG.3

FIG.4

FIG.5a

FIG.5b

FIG.5c

FIG.5d